(19) Europäisches Patentamt
European Patent Office
Office européen des brevets

(11) **EP 2 042 166 A1**

(12) **EUROPEAN PATENT APPLICATION**

(43) Date of publication:
**01.04.2009 Bulletin 2009/14**

(51) Int Cl.:
*A61K 9/51* (2006.01)          *A61K 38/00* (2006.01)

(21) Application number: **07018767.9**

(22) Date of filing: **25.09.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **The Jordanian Pharmaceutical Manufacturing Co.**
**11710 Naor (JO)**

(72) Inventors:
• **Badwan, Adnan, Dr.**
**11185 Amman (JO)**

• **Al-Remawi, Mayyas, Dr.**
**13713 Russiefa (JO)**
• **Adel Ginna, Nidal, Dr.**
**11152 Amman (JO)**
• **El-Sayed, Amani**
**11821 Amman (JO)**

(74) Representative: **Winkler, Andreas Fritz Ernst**
**Forrester & Boehmert**
**Pettenkoferstrasse 20-22**
**80336 München (DE)**

(54) **Nanocapsules for oral delivery of proteins**

(57)     The present invention relates to a novel type of nanocapsule. In addition, the present invention concerns the use of such a nanocapsule for the delivery of an active component, such as a protein or a polypeptide, such as, for example, insulin, a method for manufacturing such a nanocapsule and a pharmaceutical composition comprising such a nanocapsule.

Figure 1

EP 2 042 166 A1

**Description**

The present invention relates in general to the field of drug delivery. More precisely, the present invention relates to a novel type of nanocapsule. In addition, the present invention concerns the use of such a nanocapsule for the delivery of an active component, such as a protein or polypeptide, such as, for example, insulin, a method for manufacturing such a nanocapsule and a pharmaceutical composition comprising such a nanocapsule.

BACKGROUND OF THE INVENTION

Nanoparticles are generally defined as particles between 10 nanometers (nm) and 1000 nm. In the prior art it is known that orally administered nanoparticles can be absorbed, albeit in small quantities, not only by the way of membranous epithelial cells (M-cells) of Peyer's patches in the gut-associated lymphoid tissue, but also by the much more numerous gut enterocytes. [A. T. Florence, Nanoparticle uptake by the oral route: Fulfilling its potential? Drug Discovery Today: Technologies, Volume 2, No. 1, 2005, Pages 76-81].

Nanoparticle drug delivery utilizing degradable polymers provides a more efficient and less risky solution to many drug delivery challenges. The advantages of using these systems for drug delivery are many, wherein the most important are that these systems are absorbable, degradable and, in addition, might provide protection against the environment of the gastrointestinal tract. Thus, these systems might be useful for the oral delivery of active compounds sensitive to acid and enzymes in the human gut, specifically protein or peptide drugs, such as insulin.

Insulin, an anabolic polypeptide hormone that regulates carbohydrate metabolism, is used medically to treat some forms of diabetes mellitus. For instance, patients with type 1 diabetes mellitus depend on external insulin for their survival because of the absence of the hormone. So far, insulin cannot be taken orally. Like nearly all other proteins introduced into the gastrointestinal tract, insulin is reduced to fragments (even single amino acid components), whereupon all 'insulin activity' is lost. Thus, insulin is usually taken as subcutaneous injections by single-use syringes with needles, an insulin pump, or by repeated-use insulin pens with needles. Therefore, the oral administration of insulin would present a more convenient form of application as pain caused by the injection, stress of multiple daily injections and possible infections could be avoided which finally would lead to a higher patient compliance.

One example for a biodegradable polymer used for the fabrication of nanoparticles is the polymer called chitosan. Chitosan provides a high degree of biocompatibility for drug delivery systems. [J. Leroux, E. Allemann, R. Gurny, Biodegradable nanoparticles - From sustained release formulations to improved site specific drug delivery, J. Controlled Release, volume 39, 1996, Pages 339-350; K. Soppimath, T. Aminabhavi, A. Kulkarni, W. Rudzinski, Biodegradable polymeric nanoparticles as drug delivery devices, J. Controlled Release, volume 70, 2001, Pages 1-20; K. M. Varum, M. Myhr, R. N. Hjerde, O. Smidsrod, In vitro degradation rates of partially N-acetylated chitosans in human serum, Carbohydrate Research, Volume 299, 1997, Pages 99-101].

Chitosan is an abundant natural polymer, which is obtained by alkaline N- deacetylation of chitin. Chitosan acts as an absorption enhancer in the intestine by increasing the residence time of dosage forms at mucosal sites, inhibiting proteolytic enzymes, and in addition, increasing the permeability of proteins and peptides across mucosal membranes [R. Hejazi, and M. Amiji, 2003., J. Controlled Release, volume 89, Pages 151-165].

There have been many attempts using chitosan nanoparticles for the delivery of proteins or polypeptides, and more specifically for the delivery of insulin. One approach to formulate nano-or microparticles was by the interaction of chitosan with other polymeric materials, such as, for example, alginate, cyclodextrin or tripolyphosphates. [Zengshuan Ma, Pharmacological activity of peroral chitosan-insulin nanoparticles in diabetic rats, Int. J. Pharm. 293 (2005) 271-280; Catarina M. Silva, António J. Ribeiro, Domingos Ferreira and Francisco Veiga, Insulin encapsulation in reinforced alginate microspheres prepared by internal gelation, European Journal of Pharmaceutical Sciences, Volume 29, Issue 2, 1 October 2006, Pages 148-159; Susana Martins, Bruno Sarmento, Eliana B. Souto and Domingos C. Ferreira, Insulin-loaded alginate microspheres for oral delivery - Effect of polysaccharide reinforcement on physicochemical properties and release profile, Carbohydrate Polymers, Volume 69, Issue 4, 2 July 2007, Pages 725-731; Bruno Sarmento, Domingos Ferreira, Francisco Veiga and António Ribeiro, Characterization of insulin-loaded alginate nanoparticles produced by ionotropic pre-gelation through DSC and FTIR studies, Carbohydrate Polymers, Volume 66, Issue 1, 5 October 2006, Pages 1-7; S. Sajeesh and Chandra P. Sharma , Cyclodextrin-insulin complex encapsulated polymethacrylic acid based nanoparticles for oral insulin delivery, International Journal of Pharmaceutics, Volume 325, Issues 1-2, 15 November 2006, Pages 147-154].

    a) providing a solution comprising a hydrophilic oligopolymer,
    b) providing a solution comprising an active compound,
    c) mixing and stirring the solution of step a) with the solution of step b),
    d) providing a solution comprising fatty materials,
    e) dropping the solution of step c) into the solution of step d),

f) homogenizing the resulted mixture of step e) at a high-pressure, and

g) dropping the homogenized solution into a hydrophilic oligopolymer solution.

In a further embodiment the present invention relates to a pharmaceutical composition comprising a nanocapsule according to the present invention.

In another embodiment the present invention concerns the use of a nanocapsule according to or a pharmaceutical composition according to present invention in the manufacture of a medicament for the treatment of patients suffering from endogenous insulin secretion deficiency.

DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

As outlined above, there is still a need in the prior art to provide a new drug delivery system. In particular, it would be a great advantage to provide a drug delivery system suitable for the oral delivery of active compounds, which are sensitive to acid and enzymes of the human gut, such as proteins or polypeptides, such as, for example, insulin.

It was found, surprisingly, that the reaction of hydrophilic oligopolymers and fatty materials results in a water-insoluble matrix material, which further can be converted into a nanocapsule having a hydrophilic core and a hydrophobic shell. Surprisingly, the inclusion of a polypeptide or Another approach to formulate a nanoparticle delivery system comprising chitosan was modifying the structure of chitosan or using chitosan derivatives. [Peroral Delivery of Insulin Using Chitosan Derivatives: A Comparative Study of Polyelectrolyte Nanocomplexes and Nanoparticles, International Journal of Pharmaceutics, In Press, Accepted Manuscript, Available online 17 May 2007; Synthesis and characterization of PEG modified N-trimethylaminoethylmethacrylate chitosan nanoparticles, European Polymer Journal, In Press, Corrected Proof, Available online 4 April 2007; Lichen Yin, Likun Fei, Fuying Cui, Cui Tang and Chunhua Yin, Superporous hydrogels containing poly(acrylic acid-co-acrylamide)/O-carboxymethyl chitosan interpenetrating polymer networks, Biomaterials, Volume 28, Issue 6, February 2007, Pages 1258-1266].

Although there have been many attempts the oral delivery of most therapeutic peptides and proteins remains still an unresolved challenge mainly because of the large size, hydrophilicity and instability of these macromolecules, such as insulin (Lee, V. H.L., Yamamoto, A., 1990. Adv. Drug Deliv. Rev., 4, 171-207).

Thus, it would be a great advantage to provide a system suitable for the delivery of active compounds, which are in particular sensitive to acid and enzymes of the human gut, such as proteins or polypeptides, such as, for example, insulin.

SUMMARY OF THE INVENTION

The object of the present invention, in one preferred embodiment thereof, is solved by a nanocapsule having a hydrophilic core and a hydrophobic shell, wherein the nanocapsule is the reaction product of at least one hydrophilic oligopolymer and at least one fatty material.

In a further preferred embodiment the object of the present invention is solved by the use of a nanocapsule according to the present invention for the delivery of an active component, preferably for the delivery of a protein and/or a polypeptide.

Additionally the object of the present invention, in a preferred embodiment thereof, is solved by a method of manufacturing a nanocapsule according the present invention, comprising the steps of a protein, such as, for example insulin, in a nanocapsule according to the present invention resulted in its protection from enzymatic hydrolysis in the gastrointestinal tract after oral administration.

Therefore, in a first aspect the present invention provides a nanocapsule having a hydrophilic core and a hydrophobic shell, wherein the hydrophobic shell is the reaction product of at least one hydrophilic oligopolymer and at least one fatty material. The hydrophilic core of the nanocapsule according to the present invention gathers water-soluble materials and the hydrophobic shell provides a protection shell of encapsulated materials, such as active compounds, against aqueous medium, such as the aqueous medium of the gastrointestinal tract.

The term "capsule" as used herein refers to particles having a shell component and a core component, wherein the shell component encloses at least partially the core component.

The term "nanocapsule" as used herein refers to particles having a variety of shapes, including but not limited to spherical, elongated or even rod-like spherical shape. Preferably, the nanocapsules according to the present invention have a substantially spherical shape. In addition, the nanocapsules according to the present invention have a diameter in the range of about 5 nm to about 5000nm, preferably of about 10 nm to 1000 nm, and most preferably from about 20 nm to about 500 nm.

The conversion of a capsule into nanocapsule results in the increase of the absorption via the gastrointestinal tract besides the protection.

In one embodiment of the nanocapsule according to the present invention the hydrophilic oligopolymer is a non-ionic or cationic oligopolymer and the at least one fatty material is anionic. Preferably the hydrophilic oligopolymer is a cationic oligopolymer. More preferably, the hydrophilic oligopolymer consists essentially of a polymeric backbone having a low molecular weight and cationic properties.

In one preferred embodiment of the nanocapsule according to the present invention the hydrophilic oligopolymer is water-soluble.

In one embodiment of the nanocapsule according to the present invention the hydrophilic oligopolymer is chitin, chitosan material or derivatives thereof. Preferably the hydrophilic oligopolymer is chitosan material.

In a preferred embodiment of the nanocapsule according to the present invention the chitosan material has a molecular weight in the range of about 1 kDa to about 100 kDa.

In a further preferred embodiment of the nanocapsule according to the present invention the chitosan material has a molecular weight in the range of about 2 kDa to about 30 kDa. The molecular weight of chitosan can be controlled by varying the number of basic monomeric glucosamine units. Chitosan material having a low molecular weight has preferred absorption characteristics.

In one embodiment of the nanocapsule according to the present invention the chitosan material has a degree of deacetylation in the range of about 50 to about 100%.

In a preferred embodiment of the nanocapsule according to the present invention the chitosan material has a degree of deacetylation in the range of about 80 to about 100%. The primary amine functional group of the chitosan material gives the interaction characteristics of the oligopolymer toward the acidic moieties. Since the primary amine functional group of the chitosan material might be acetylated, the degree of deacetylation influences the interaction characteristics toward the acidic moieties.

In one embodiment of the nanocapsule according to the present invention the chitosan material is selected from the group consisting of chitosan, chitosan derivatives, base oligochitosan amine, and salts thereof.

In another embodiment of the nanocapsule according to the present invention the hydrophilic oligopolymer further comprise polymeric structures selected from the group consisting of deacetylated hyaluronic acid, polylysine, and polyamines. It is known in the prior art that these polymeric structures have the ability to complex with negatively charged structures.

In one embodiment of the nanocapsule according to the present invention the fatty material is selected from the group consisting of fatty acids, triglycerides, vegetable oils, fats, and waxes.

In a preferred embodiment of the nanocapsule according to the present invention the vegetable oil is selected from the group consisting of olive oil, corn oil, cottonseed oil, and soybean oil.

In another preferred embodiment of the nanocapsule according to the present invention the fatty acid is selected from the group consisting of pentanoic acid, hexanoic acid, decanoic acid, lauric acid, palmitic acid, stearic acid, oleic acid or salts thereof. It is known in the prior art that these fatty acids, either saturated or unsaturated, are capable to interact with the oligopolymers used in the present invention.

In a further preferred embodiment of the nanocapsule according to the present invention the fatty acid is sodium lauryl sulfate (SLS) or sodium oleate.

In a preferred embodiment of the nanocapsule according to the present invention the hydrophilic oligopolymer is a water-soluble polymeric structure, such as chitosan, having monomer units <100 units per polymer and being capable to interact with negatively charged water-soluble fatty acids or fatty acid derivatives, such as sodium lauryl sulfate. The resultant complex forms a water-insoluble system having a hydrophilic core and a hydrophobic surface. In order to convert the water-insoluble system into a nanocapsule system having a hydrophilic core and a hydrophobic surface any suitable method known in the prior art, such as high-pressure homogenization, can be applied during the complexation process.

In one embodiment of the nanocapsule according to the present invention the mole ratio between chitosan material and fatty acid or the salt thereof is between 1:10 to 10:1 (based on moles of glucosamine monomer units).

In a preferred embodiment of the nanocapsule according to the present invention the mole ratio between chitosan material and fatty acid or the salt thereof is 1:5 (based on moles of glucosamine monomer units).

In one embodiment of the nanocapsule according to the present invention interaction between an oligopolymer and a fatty material is achieved upon mixing, which finally results in an aggregated water-insoluble material. Preferably, such interaction is an ionic interaction as achieved, for example, by mixing cationic chitosan polymer with a fatty acid salt. Alternatively, the interaction is an electrostatic interaction, which can be achieved by the interaction of non-ionic species of the base oligochitosan amine and the fatty acid in its acid form. Further, the interaction can be also achieved upon the interaction of non-ionic species of the base oligochitosan amine and the fatty acid in its acid form to form a covalent link using any methodology known in the prior art for such conjugation. The preferred water-soluble polycationic oligopolymers interaction with anionic fatty acids permits the efficient encapsulation and low particle size.

In a preferred embodiment of the nanocapsule according to the present invention interaction between a cationic hydrophilic oligopolymer, such as chitosan, its salts or derivatives, and anionic fatty acids, their salts or their derivatives, such as sodium lauryl sulfate and oleic acid, is achieved by ionic or covalent interactions.

In another embodiment of the nanocapsule according to the present invention the hydrophobic shell further comprises at least one component that is pH insensitive and that is indigestible by enzymes present in the mammalian intestinal tract. This embodiment of the nanocapsule according to the present invention is particular suitable to be administered orally or rectally. The pH insensitive material suitable to be used according to the present invention is selected from the group consisting of chitin, chitosan, fatty acids, triglycerides, vegetable oils, fats, waxes, and derivatives thereof.

In one embodiment of the nanocapsule according to the present invention the hydrophobic shell is resistant to dissolution or acidic degradation at pH levels lower than 5.

In a preferred embodiment of the nanocapsule according to the present invention the hydrophobic shell is resistant to dissolution or acidic degradation at pH levels lower than 4. Preferably, the nanocapsule according to the present invention comprises a water-insoluble matrix composed of a chitosan fatty acid complex that is resistant to dissolution or acidic degradation at pH levels found in the stomach, which pH is lower than about 4.

In one embodiment of the nanocapsule according to the present invention the hydrophobic shell further comprises at least one surfactant.

In a preferred embodiment of the nanocapsule according to the present invention the at least one surfactant is in the range of about 0.01 to about 30 % w/w of the nanocapsule.

In a further preferred embodiment of the nanocapsule according to the present invention the at least one surfactant is in the range of about 1 to about 10 % w/w of the nanocapsule.

In one preferred embodiment of the nanocapsule according to the present invention the at least one surfactant is selected from the group consisting of sodium lauryl sulfate, sorbitan ester and polysorbates.

In one embodiment of the nanocapsule according to the present invention the hydrophilic core comprises an active compound. The active compound encapsulated by the nanocapsule can be present in any form, including but not limited to solid forms, such as nanoparticles, crystalline forms, amorphous forms, and liquid forms.

In a preferred embodiment of the nanocapsule according to the present invention the active compound is a protein or a polypeptide.

In a further preferred embodiment of the nanocapsule according to the present invention the polypeptide is insulin. Any type of insulin known in the art to be suitable for the treatment of an insulin secretion deficiency, such as diabetics, can be used in the present invention. The insulin can be either of animal or human origin, such as, for example, recombinant human insulin. Further, the insulin can be selected from the group consisting of quick-acting, short-acting, immediate-acting or long-acting insulin analogs. Preferably, recombinant human insulin powder having short-acting properties is used.

"Quick-acting", as used herein, refers to an insulin analog, which begins to work within 5 to 15 minutes and which is active for 3 to 4 hours. "Short-acting", as used herein, refers to an insulin analog, such as regular insulin, which starts to work within 30 minutes and which is active about 5 to 8 hours. "Intermediate-acting", as used herein, refers to an insulin analog, such as NPH or lente insulin, which starts to work within 1 to 3 hours and which is active 16 to 24 hours. "Long-acting", as used herein, refers to an insulin analog, such as ultralente insulin, which starts to work within 4 to 6 hours and which is active 24 to 28 hours.

In another embodiment of the nanocapsule according to the present invention the nanocapsule further comprises water-soluble bioenhancers, anti-oxidants, mineral buffering agents, flavoring agents, or sweetening agents.

In one embodiment the nanocapsule according to the present invention is encapsulated, coated, or surrounded by at least one additional material.

In one preferred embodiment the nanocapsule according to the present invention the at least one additional material contains lipophilic moieties leading to a nanocapsule exhibiting low solubility properties. Most preferably the material is a fatty material having an anionic tendency at high pH values, such as a fatty acid salt. The lipophilic moieties enhance the absorption and the protection of the gastro labile drugs.

In one embodiment the nanocapsule according to the present invention is capable of an in vitro or in vivo release of about 70 to about 100 percent of the core material over a period of about 4 to about 48 hours, preferably over a period of about 12 to 24 hours.

The nanocapsule according to the present invention is suitable to deliver a poor bioavailable drug such as proteins or polypeptides exhibiting low permeability across the membrane of the subject to which the drug is administered. Thus, the nanocapsule according to the present invention is suitable to deliver active compounds having a low permeability and/or a low solubility.

In another aspect the present invention concerns the use of a nanocapsule according to the present invention for the delivery of an active component. The nanocapsule delivery system according to the present invention provides a stable and protective nanocarrier until the system is activated in order to release the active compound by means of one or more dissolution mechanisms. Thus, the use of a nanocapsule according to the present invention allows the oral delivery of drugs sensitive to acid and enzymes in the human gut, such as a polypeptide or a protein.

In one embodiment of the use according to the present invention, the active compound is a protein or a polypeptide.

In a preferred embodiment of the use according to the present invention, the polypeptide is insulin.

The nanocapsule according to the present invention can be used in any suitable pharmaceutical dosage form, for example, compressed in form of a tablet, in form of multi-particulates filled in a capsule or suspended in a liquid carrier, in order to obtain either an immediate release or sustained release properties.

In a third aspect the present invention relates to a method of manufacturing a nanocapsule according the present invention, comprising the steps of

a) providing solution comprising a hydrophilic oligopolymer,
b) providing a solution comprising an active compound,
c) mixing and stirring the solution of step a) with the solution of step b),
d) providing a solution comprising fatty materials,
e) dropping the solution of step c) into the solution of step d),
f) homogenizing the resulted mixture of step e) at a high-pressure, and
g) dropping the homogenized solution into a hydrophilic oligopolymer solution.

In one embodiment of the method according to the present invention is the hydrophilic oligopolymer of step a) a cationic hydrophilic polymer, such as, for example, ionized chitosan.

In another embodiment of the method according to the present invention is the active compound of step b) a protein or a polypeptide.

In a further embodiment of the method according to the present invention is polypeptide insulin, such as, for example, ionized insulin.

In one embodiment of the method according to the present invention homogenization of step (f) is performed at a pressure in the range of 500-2500 bar.

In a preferred embodiment of the method according to the present invention the method comprises the treatment of a mixture of an active compound and a chitosan oligopolymer, and the addition of the salt of a fatty acid in order to form a water-insoluble matrix material, which can be converted into a nanocapsule system with the aid of high-pressure homogenization. The preferred process of the manufacture of a nanocapsule according to the present invention is the use of high-pressure homogenization. However, other any methods suitable for the formulation of nanocapsules and known in the art can be used.

In another aspect the present invention concerns a pharmaceutical composition comprising a nanocapsule according to the present invention.

In one embodiment the pharmaceutical composition according to the present invention comprises a nanocapsule consisting essentially of a core containing an active compound and a shell surrounding the core, wherein the nanocapsule has both a protecting and an absorption-enhancing activity of poorly bioavailable active compounds, such as proteins or polypeptides.

In one embodiment the pharmaceutical composition according to the present invention further comprises one or more of fillers, diluents, excipients and materials having an effect on the release properties of the active compound.

In one preferred embodiment the pharmaceutical composition according to the present invention comprises a nanocapsule according to the present invention encapsulating insulin.

The pharmaceutical composition according to the present invention is administered by contacting it with a body membrane capable of absorbing the active compound. The contacted membrane can be any body membrane via which the active compound can be absorbed, including but not limited to membranes of the gastrointestinal tract, nasal passages, rectum, pulmonary alveoli, eye or buccal cavity. A particularly preferred membrane is found within the gastrointestinal tract.

In one preferred embodiment the nanocapsule and the pharmaceutical composition according to the present invention release the active compound after passing through the small intestine. The nanocapsule and the pharmaceutical composition according to the present invention are capable to release an enhanced amount of the active compound and to increase the bioavailability of the active compound in a continuous, sustained and controlled release rate.

In one embodiment the pharmaceutical composition according to the present invention comprise a vehicle, wherein the nature of the vehicle depends on the form of the pharmaceutical composition.

The term "vehicle" in the sense of the present invention is broadly used and includes various types of pharmaceutically acceptable ingredients other than the active compound. Examples of vehicles include fillers, diluents, excipients and materials, which have an effect on the release properties of the active compound, for example, control-release materials.

In another embodiment the pharmaceutical composition according to the present invention comprises a vehicle in order to aid the delivery of the nanocapsules via other non-oral route of administrations for example, rectal, nasal, transdermal, intraocular and parenteral the composition of which will depend on the dosage form, the route of administration and intended purpose of treatment.

In a further aspect the present invention concerns the use of a nanocapsule or a pharmaceutical composition according to present invention in the manufacture of a medicament for the treatment of patients suffering from endogenous insulin secretion deficiency. Preferably, the nanocapsule or the pharmaceutical composition according to present invention are used in the manufacture of a medicament which is administered orally.

In one embodiment of the use according to the present invention the patients are diabetic patients. The nanocapsule or the pharmaceutical composition according to the present invention are suitable for the oral delivery of exogenous insulin via the oral route of patients suffering from endogenous insulin secretion deficiency, such as, for example diabetic patients.

Another aspect of the present invention relates to a method of treatment of diabetics comprising administering therapeutically effective amount of a nanocapsule or a pharmaceutical composition according to the present invention comprising insulin.

BRIEF DESCRIPTION OF THE DRAWINGS

Figure 1 shows the FTIR of AI) sodium lauryl sulfate (SLS), AII) chitosan with an average molecular weight of 2.3 KDa, BIII) chitosan SLS physical mixture, and BIV) chitosan SLS complex.

Figure 2 shows the average hydrodynamic diameter of nanoparticles containing insulin prepared according to example 1.

Figure 3 shows the average zeta potential of nanoparticles containing insulin prepared according to example 1.

Figure 4 shows the average glucose level for each group prior inclusion of the groups in the in vivo study. Error bars represent standard deviations.

Figure 5 shows the percent decrease of the glucose level time profile for STZ diabetic rats given subcutaneous insulin and insulin-chitosan complex using two chitosan molecular weights 2.3 KDa and 30 KDa in a dose of 1 U/kg, n=4, error bars represent the standard error of the mean (SEM).

Figure 6 shows the percent decrease in glucose level time profile for STZ diabetic rats given a formula of oral insulin-chitosan 2.3KD and SLS complex in a dose of 50U/kg, n=5, error bars represent SEM.

EXAMPLES

**Example 1**

Determination of the molecular weight and the degree of deacetylation of the chitosan oligosaccharide

The viscosity average molecular weight of chitosan was calculated using the Mark-Houwink equation as described below.

$$[\eta] = K\left(M_v\right)^a$$

where $[\eta]$ is the intrinsic viscosity of the chitosan.

The intrinsic viscosity of chitosan in deionized water was measured using a vibroviscometer VS 10-Japan in a water bath at a constant temperature of 25°C. K and a are constants for the given solute-solvent system and temperature. For chitosan, the values of K and a are influenced by the degree of deacetylation, pH and ionic strength of the solvent.

They were determined based on a recent calculation method [Mohammad Kasaai, Carbohydrate Polymers, volume 68, 2007, Pages 477-488].

The degree of deacetylation of chitosan was determined using the UV spectrophotometric method according to the British Pharmacopoeia (BP) 2004. In table 1 the results of the average molecular weight and the degree of deacetylation are summarized. Two grades of low molecular weight chitosan <50 KD with nearly the same degree of deacetylation were investigated.

**Table 1: Summary of average molecular weight determined by viscosity and degree of deacetylation determined by UV method.**

| Polymer | Source | Average molecular weight | Degree of deacetylation |
|---|---|---|---|
| Chitosan I | BN NC041201, NiceChem Co. Ltd, 5-102 No. 599 Yixian, RD, Shanghai, China | 2.3 KDa | 85 |
| Chitosan 2 | BN NC060826, NiceChem Co. Ltd, 5-102 No. 599 Yixian, RD, Shanghai, China | 30 KDa | 86 |

**Example 2**

Chitosan saccharide interaction with fatty acid derivative such as sodium lauryl sulfate (SLS) and Characterization of chitosan SLS complex via FTIR (Fourier Transform Infrared Spectroscopy)

The chitosan oligosaccharidel of example 1 (6% w/v) was dissolved in deionized water and the pH was increased up to pH 7 using 0.2 M NaOH (solution 1). Then sodium lauryl sulfate (1% w/v) was dissolved in water and 0.05 M KH2PO4 was added to raise the pH up to 5 (solution 2). 3 ml of solution 1 was slowly dropped with vigorous mixing to 20 ml of solution 2. Precipitation occurred and the particles were filtered out and freeze dried prior the FTIR measurements. The physical mixture was used for comparison purposes prepared in the same ratios.

An appropriate amount of the sample was mixed with pure KBr using mortar and pestle and then compressed into discs. The disc was placed on the FTIR holder and the diffraction of IR spectra was measured. The results were compared with reference to a physical mixture.

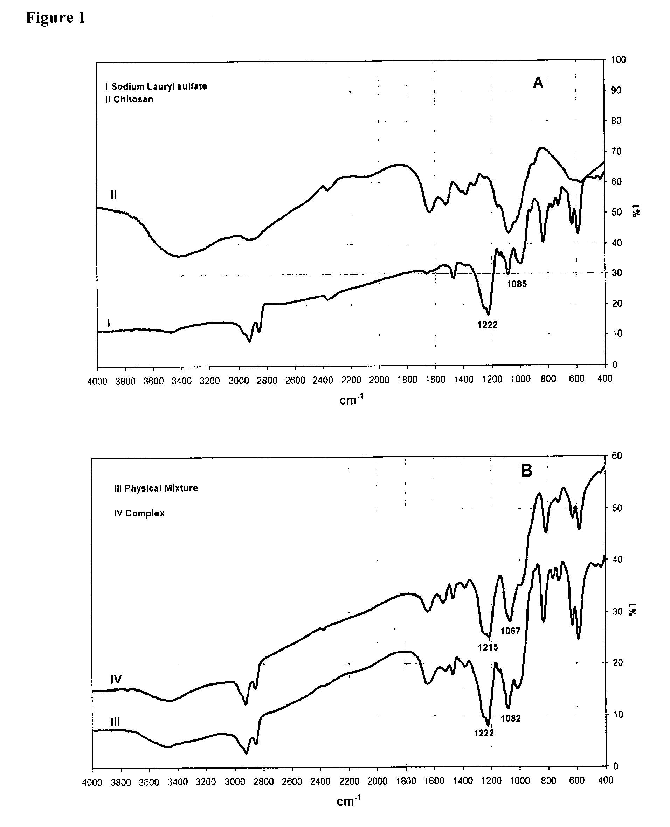

To confirm the complex formation of SLS with chitosan through ionic pairing, the precipitates obtained upon addition of SLS to chitosan solution were analyzed using FTIR. The peaks of $SO_2$ stretch (asymmetric 1222 cm$^{-1}$, and symmetric 1085 cm$^{-1}$) in the complex were shifted to the lower field relative to unbound SLS (1215 and 1067 cm$^{-1}$), which is attributed to the salt formation of sulfate groups as shown in Figure 1.

**Example 3**

Preparation of nanoparticles

To enhance the formation of the polyelectrolyte complex between chitosan and insulin, both of them have to be ionized in order to bear opposite charges. Insulin powder was dissolved in 0.1 M HCl and the pH of this solution was adjusted to 7 with tris buffer. Insulin carries a negative charge above its isoelectric point (5.3). Chitosan powder oligosaccharide1 of example 1 was dissolved in water and its pH was adjusted to 5.5 with NaOH (0.2 M). At this pH chitosan is protonated (the pKa of chitosan is about 6.5). Equal volumes of the polymer and insulin were stirred gently for 15 min. Different polymer insulin ratios were prepared.

The gelation of chitosan upon the addition of sodium lauryl sulfate (SLS) was utilized in order to form microparticles. All SLS has to be consumed, why the numbers of mmole of both compounds have to be equal.

A calculated volume of the insulin-chitosan complex was dropped into 20 ml of SLS solution (1% pH 5 adjusted with $NaH_2PO_4$) stirred with a magnetic stirrer. The microparticles were homogenized at 1000 bar (5 cycles). The homogenized formula was dropped into chitosan solution (6% pH 5.5) stirred at 300 rpm.

**Example 4**

Encapsulation Efficiency

The amount of insulin entrapped within nanoparticles prepared according to example 1 was determined indirectly, i.e., by measuring the amount of free insulin with a RP-HPLC assay in the supernatant recovered after centrifugation at 11,000 rpm for 50 min at 5 °C. The drug entrapment was expressed as the percentage of the insulin difference between the total amount (obtained by solubilization of nanoparticles in 1% w/v of Tween 80) and the free amount in the supernatant relative to the total amount.

$$\text{Encapsulation efficiency} = \frac{\text{total amount of insulin - free amount of insulin}}{\text{total amount of insulin}} X \ 100$$

Analytical determination of rh-insulin by high pressure liquid chromatography (HPLC) was performed at 40 °C as described in the USP method. The conditions employed were: column, ACE 5 $\mu$m, 250x 5 mm i.d; detection at 24 nm; eluent, acetonitrile-aqueous solution of 0.2 M sodium sulfate acidified with concentrated phosphoric acid pH 2.3 (volume ratio 26:74). The flow rate was 1 ml/min. Linearity was demonstrated by construction of a calibration curve for low concentrations of insulin (0.1, 0.2, 0.3, 0.5 and 1U/ml). The encapsulation efficiency based on data of Table 2 can be estimated to be 83%.

**Table 2: HPLC response for insulin standard and sample before and after centrifugation**

| Name | Response | % Insulin |
|------|----------|-----------|
| Standard (1U/ml) | 1311802 | 100 |
| Sample assay before centrifugation (Total assay) (1U/ml) | 1182602 | 90 |
| Sample assay of supernatant after centrifugation (1 U/ml) | 205502 | 15 |

**Example 5:**

Immunological activity evaluation of encapsulated insulin by ELISA

The immunological activity of encapsulated insulin prepared according to example 3 before and after incubation with simulated gastric fluid (SGF) without pepsin enzyme for 1 hr was assessed using ELISA technique. The nanocapsules were dissolved in a 1:1 mixture of methanol and 0.01 M HCl containing 1% Tween 80 and vortexed for 1 min. The nanocapsules incubated with SGF were first separated by centrifugation at 11000 rpm for 50 min at 5 °C, then dissolved in a 1:1 v/v mixture of methanol and 0.01 M HCl containing 1% Tween 80 and vortexed for 1 min. The insulin content was analyzed by ELISA according to the instructions of the manufacture (Ins-EASIA, Biosource Europe SA., Rue de l'Industrie,8, B-1400 Nivelles, Belgium).

Table 3 shows that the insulin was preserved after exposure to SGF when compared to standard insulin. In addition, insulin formulated was extracted and assayed using the ELISA Kit showing a value nearly close to the standard value. This means that the immunological activity of insulin was almost completely preserved under the experimental conditions and applied formulation procedure.

**Table 3: Analysis of insulin formula after exposure to SGF for 1 hr using ELISA-insulin Kit.**

| Name | Concentration (IU/ml) |
|------|----------------------|
| Insulin Standard | 1.16784 |
| Total assay | 1.36464 |
| SGF 1hr sample 1 | 1.23624 |
| SGF 1hr sample2 | 1.08704 |

**Example 6:**

Determination of the particle size and the zeta potential of nanocapsules

The particle size distribution and zeta potential of the dispersed nanocapsules as described in example 3 was determined by dynamic light scattering at an angle of 173° at 25 °C, using a Malvern Zetasizer Nano ZS (Malvern, Worcestershire, UK). Samples were measured in triplicates using the refractive index and viscosity of pure water in calculations. The zeta potential was measured with laser Dopler velocimetry at 25 °C on the same instrument and with the same viscosity and dielectric constant pure water for calculations. For both of the above measurements, nanoparticles were diluted 1:10 v/v with pure dust free deionized water.

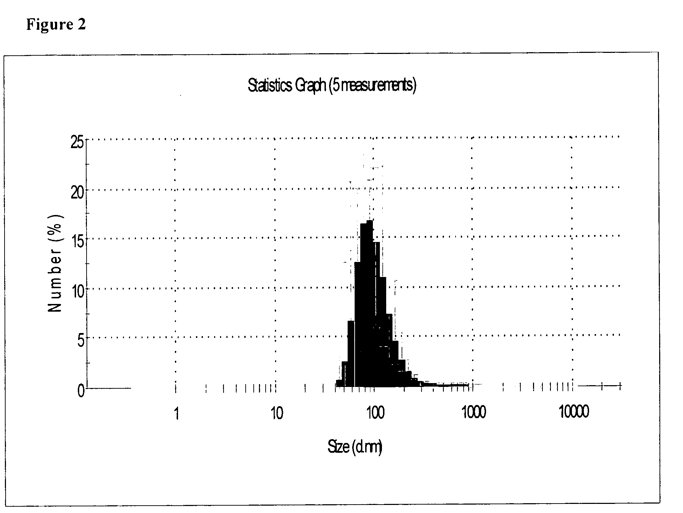

The average hydrodynamic diameter (%Number) was around 100 nm as shown in Figure 2. This suggests that the nanocapsules are present in a very small size that might facilitate the their penetration through the gastrointestinal tract (GIT).

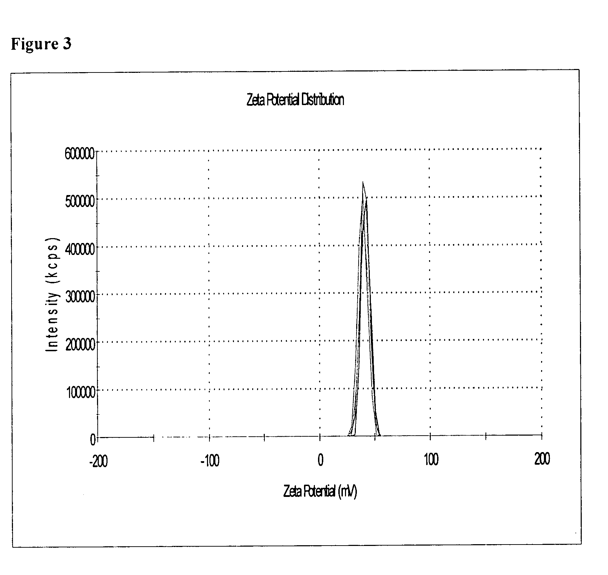

The zeta potential was +40.8 mV as shown in Figure 3. This suggests the presence of positively charged nanocapsules that may aid in their physical stability and also may increase their permeability through the GIT.

**Example 7:**

In-Vivo Study

Male Wistar rats (weighing 200-300 g) were made diabetics by intraperitonial injections of streptozotocin (45 mg/kg) dissolved in citrate buffer pH 4.5. They were considered to be diabetics when the base line glucose levels were above 150mg/dl. The diabetic rats were fasted overnight. They had free access to water. Animals were divided into 3 groups.

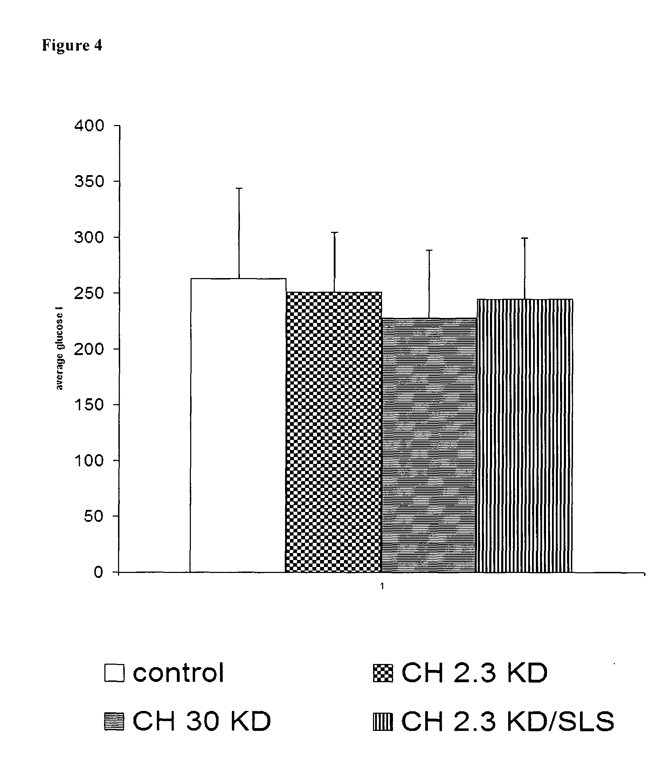

The average glucose level for each group prior inclusion of the groups in the in vivo study are shown in Figure 4. The average glucose level was above 150 mg/dl.

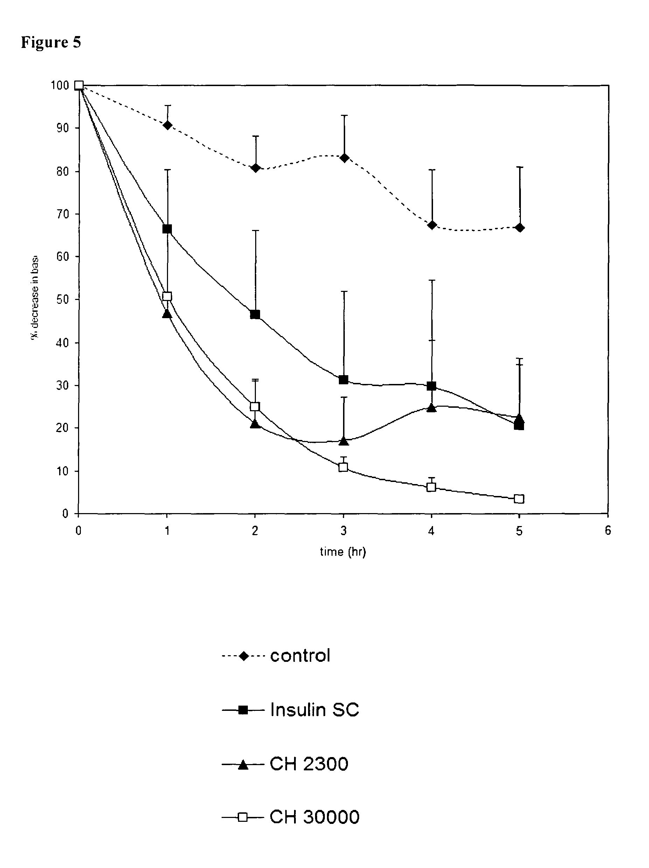

To evaluate insulin bioactivity upon complexing with chitosan 1 and 2 of example 1, native insulin and insulin complexed with chitosan of two molecular weights 2.3 KD and 30 KD were injected to diabetic rats in a dose of 1 U/kg a control group of STZ diabetic rats (not treated) was used to monitor the glucose baseline. Figure 5 indicates that insulin complex is almost as potent as free insulin.

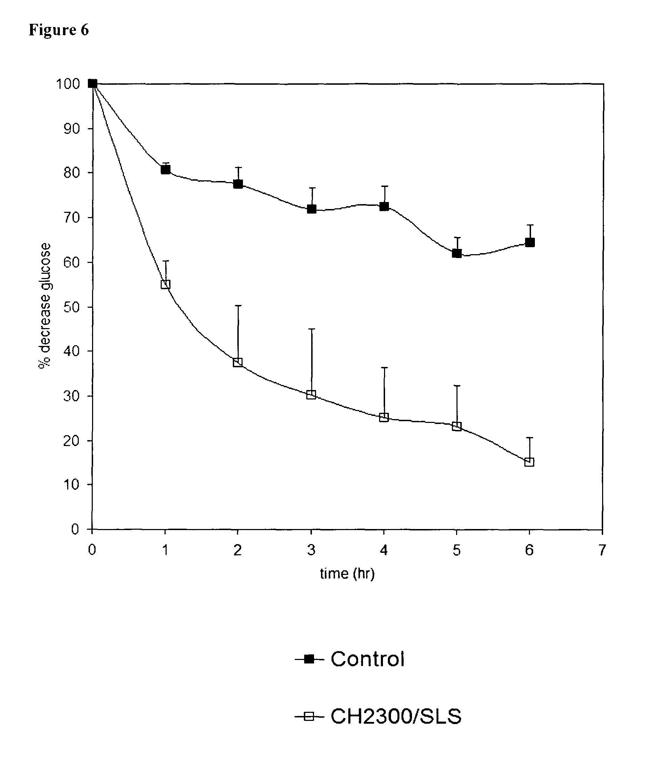

In order to evaluate insulin complexed with chitosan of molecular weight 2.3 KDa (prepared as in example 3), diabetic rats were divided into 2 groups: Group 1 (control group) were given oral insulin solution (50 U/kg), group 2 were given oral insulin formula (50 U/kg). Figure 6 shows that oral insulin formula resulted in a significant decrease in glucose level.

**Claims**

1.   Nanocapsule having a hydrophilic core and a hydrophobic shell, wherein the nanocapsule is the reaction product of at least one hydrophilic oligopolymer and at least one fatty material.

2.   Nanocapsule according to claim 1, wherein said hydrophilic oligopolymer is a non-ionic or cationic oligopolymer and wherein said at least one fatty material is anionic.

3.   Nanocapsule according to claim 1 or 2, wherein said hydrophilic oligopolymer is chitin, chitosan material or derivatives thereof.

4.   Nanocapsule according to claim 3, wherein said chitosan material has a molecular weight of about 1 kDa to about 100 kDa.

5.   Nanocapsule according to claim 4, wherein said chitosan material has a molecular weight of about 2 kDa to about 30 kDa.

6.   Nanocapsule according to any of claims 3 to 5, wherein said chitosan material has a degree of deacetylation in the range of about 50 to about 100%.

7.   Nanocapsule according to claim 6, wherein said chitosan material has a degree of deacetylation in the range of about 80 to about 100%.

8. Nanocapsule according to any of claims 3 - 7, wherein said chitosan material is selected from the group consisting of chitosan, chitosan derivatives, base oligochitosan amine, and salts thereof.

9. Nanocapsule according to any of claims 1 - 8, wherein said hydrophilic oligopolymer further comprises polymeric structures selected from the group consisting of deacetylated hyaluronic acid, polylysine, and polyamines.

10. Nanocapsule according to any of claims 1 - 9, wherein said fatty material is selected from the group consisting of fatty acids, triglycerides, vegetable oils, fats, and waxes.

11. Nanocapsule according to claim 10, wherein the vegetable oil is selected from the group consisting of olive oil, corn oil, cottonseed oil, and soybean oil.

12. Nanocapsule according to claim 10, wherein said fatty acid is selected from the group consisting of pentanoic acid, hexanoic acid, decanoic acid, lauric acid, palmitic acid, stearic acid, oleic acid or salts thereof.

13. Nanocapsule according to claim 12, wherein said fatty acid is sodium lauryl sulfate or sodium oleate.

14. Nanocapsule according to any of claims 1 - 13, wherein the mole ratio between chitosan material and fatty acid or the salt thereof is between 1:10 to 10:1.

15. Nanocapsule according to claim 14, wherein the mole ratio between chitosan material and fatty acid or the salt thereof is 1:5.

16. Nanocapsule according to any of claims 1 - 15, wherein the hydrophobic shell comprises at least one component that is pH insensitive and that is indigestible by enzymes present in the mammalian intestinal tract.

17. Nanocapsule according to any of claims 1 - 16, wherein the hydrophobic shell is resistant to dissolution or acidic degradation at pH levels lower than 5.

18. Nanocapsule according to claim 17, wherein the hydrophobic shell is resistant to dissolution or acidic degradation at pH levels lower than 4.

19. Nanocapsule according to any of claims 1 - 18, wherein the hydrophobic shell further comprises at least one surfactant.

20. Nanocapsule according to claim 19, wherein the concentration of the at least one surfactant is in the range of about 0.01 to about 30 % w/w of the nanocapsule.

21. Nanocapsule according to claim 20, wherein the concentration of the at least one surfactant is in the range of about 1 to about 10 % w/w of the nanocapsule.

22. Nanocapsule according to any of claims 19 to 21, wherein the at least one surfactant is selected from the group consisting of sodium lauryl sulfate, sorbitan ester and polysorbates.

23. Nanocapsule according to any of claims 1 - 22, wherein the hydrophilic core comprises an active compound.

24. Nanocapsule according to claim 23, wherein the active compound is a protein or a polypeptide.

25. Nanocapsule according to claim 24, wherein the polypeptide is insulin.

26. Nanocapsule according to any of claims 1 - 25, wherein the nanocapsule further comprises water-soluble bioenhancers, anti-oxidants, mineral buffering agents, flavoring agents, or sweetening agents.

27. Nanocapsule according to any of claims 1 - 26, wherein the nanocapsule is further encapsulated, coated, or surrounded by at least one additional material.

28. Nanocapsule according to claim 27, wherein the at least one additional material contains lipophilic moieties.

29. Use of a nanocapsule according to any of claims 1 - 28 for the delivery of an active compound.

30. Use according to claim 29, wherein the active compound is a protein or a polypeptide.

31. Use according to claim 30, wherein the polypeptide is insulin.

32. Method of manufacturing a nanocapsule according to any of claims 1 - 28, comprising the steps of

    h) providing a solution comprising a hydrophilic oligopolymer,
    i) providing a solution comprising an active compound,
    j) mixing and stirring the solution of step a) with the solution of step b),
    k) providing a solution comprising fatty materials,
    l) dropping the solution of step c) into the solution of step d),
    m) homogenizing the resulted mixture of step e) at high-pressure, and
    n) dropping the homogenized solution into a hydrophilic oligopolymer solution.

33. Method according to claim 32, wherein the hydrophilic oligopolymer of step a) is a cationic hydrophilic oligopolymer.

34. Method according to claim 32 or 33, wherein the active compound of step b) is a protein or polypeptide.

35. Method according to claim 34, wherein the polypetide is insulin.

36. Method according to any of claims 32 to 35, wherein homogenization of step f) is performed at 1000 bar.

37. Pharmaceutical composition comprising a nanocapsule according to any of claims 1-28.

38. Pharmaceutical composition according to claim 37, further comprising one or more of fillers, diluents, excipients and materials having an effect on the release properties of the active compound.

39. Use of a nanocapsule according to any of claims 1 - 28 or a pharmaceutical composition according to claims 37 or 38 in the manufacture of a medicament for the treatment of patients suffering from endogenous insulin secretion deficiency.

40. Use according to claim 39, wherein the patients are diabetic patients.

**Figure 1**

Figure 2

Statistics Graph (5 measurements)

Figure 3

Figure 4

□ control          ▨ CH 2.3 KD

▤ CH 30 KD      ⦙ CH 2.3 KD/SLS

**Figure 5**

**Figure 6**

**European Patent Office**

**EUROPEAN SEARCH REPORT**

Application Number

EP 07 01 8767

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 797 870 A (JORDANIAN PHARMACEUTICAL MFG C [JO]) 20 June 2007 (2007-06-20) * examples 1,2 * * claims 1-10 * * paragraph [0003] * ----- | 1-40 | INV. A61K9/51 A61K38/00 |
| X | EP 1 834 635 A (ADVANCED IN VITRO CELL TECHNOL [ES]) 19 September 2007 (2007-09-19) * claims 1-17 * * paragraph [0023] - paragraph [0068] * ----- | 1-40 | |
| X | US 2007/116771 A1 (SUNG HSING-WEN [TW] ET AL) 24 May 2007 (2007-05-24) * claims 1-20 * * examples 1-10 * ----- | 1-40 | |
| A | DE 103 07 016 A1 (BEIERSDORF AG [DE]) 16 September 2004 (2004-09-16) * claims 1-11 * * example 1 * ----- | 1-40 | TECHNICAL FIELDS SEARCHED (IPC) |
| A | WO 97/47323 A (ZONAGEN INC [US]) 18 December 1997 (1997-12-18) * claims 1-25 * * example 5 * ----- | 1-40 | A61K |

The present search report has been drawn up for all claims

| Place of search | Date of completion of the search | Examiner |
|---|---|---|
| Munich | 12 March 2008 | Schifferer, Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 2 042 166 A1

**ANNEX TO THE EUROPEAN SEARCH REPORT
ON EUROPEAN PATENT APPLICATION NO.**

EP 07 01 8767

12-03-2008

| Patent document cited in search report | | Publication date | Patent family member(s) | | Publication date |
|---|---|---|---|---|---|
| EP 1797870 | A | 20-06-2007 | WO | 2007068311 A1 | 21-06-2007 |
| EP 1834635 | A | 19-09-2007 | WO | 2007104732 A2 | 20-09-2007 |
| US 2007116771 | A1 | 24-05-2007 | NONE | | |
| DE 10307016 | A1 | 16-09-2004 | NONE | | |
| WO 9747323 | A | 18-12-1997 | AU | 3481497 A | 07-01-1998 |
| | | | CA | 2228251 A1 | 18-12-1997 |
| | | | CN | 1198100 A | 04-11-1998 |
| | | | EP | 0843559 A2 | 27-05-1998 |
| | | | JP | 10513202 T | 15-12-1998 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. T. FLORENCE.** Nanoparticle uptake by the oral route: Fulfilling its potential?. *Drug Discovery Today: Technologies,* 2005, vol. 2 (1), 76-81
- *J. Controlled Release,* 1996, vol. 39, 339-350
- **K. SOPPIMATH ; T. AMINABHAVI ; A. KULKARNI ; W. RUDZINSKI.** Biodegradable polymeric nanoparticles as drug delivery devices. *J. Controlled Release,* 2001, vol. 70, 1-20
- **K. M. VARUM ; M. MYHR ; R. N. HJERDE ; O. SMIDSROD.** In vitro degradation rates of partially N-acetylated chitosans in human serum. *Carbohydrate Research,* 1997, vol. 299, 99-101
- **R. HEJAZI ; M. AMIJI.** *J. Controlled Release,* 2001, vol. 89, 151-165
- **ZENGSHUAN MA.** Pharmacological activity of peroral chitosan-insulin nanoparticles in diabetic rats. *Int. J. Pharm,* 2005, vol. 293, 271-280
- **CATARINA M. SILVA ; ANTÓNIO J. RIBEIRO ; DOMINGOS FERREIRA ; FRANCISCO VEIGA.** Insulin encapsulation in reinforced alginate microspheres prepared by internal gelation. *European Journal of Pharmaceutical Sciences,* 01 October 2006, vol. 29 (2), 148-159
- **SUSANA MARTINS ; BRUNO SARMENTO ; ELIANA B. SOUTO ; DOMINGOS C. FERREIRA.** Insulin-loaded alginate microspheres for oral delivery - Effect of polysaccharide reinforcement on physicochemical properties and release profile. *Carbohydrate Polymers,* 02 July 2007, vol. 69 (4), 725-731

- **BRUNO SARMENTO ; DOMINGOS FERREIRA ; FRANCISCO VEIGA ; ANTÓNIO RIBEIRO.** Characterization of insulin-loaded alginate nanoparticles produced by ionotropic pre-gelation through DSC and FTIR studies. *Carbohydrate Polymers,* 05 October 2006, vol. 66 (1), 1-7
- **S. SAJEESH ; CHANDRA P. SHARMA.** Cyclodextrin-insulin complex encapsulated polymethacrylic acid based nanoparticles for oral insulin delivery. *International Journal of Pharmaceutics,* 15 November 2006, vol. 325 (1-2), 147-154
- Peroral Delivery of Insulin Using Chitosan Derivatives: A Comparative Study of Polyelectrolyte Nanocomplexes and Nanoparticles. *International Journal of Pharmaceutics,* 17 May 2007
- Synthesis and characterization of PEG modified N-trimethylaminoethylmethacrylate chitosan nanoparticles. *European Polymer Journal,* 04 April 2007
- **LICHEN YIN ; LIKUN FEI ; FUYING CUI ; CUI TANG ; CHUNHUA YIN.** Superporous hydrogels containing poly(acrylic acid-co-acrylamide)/O-carboxymethyl chitosan interpenetrating polymer networks. *Biomaterials,* February 2007, vol. 28 (6), 1258-1266
- **LEE, V. H.L. ; YAMAMOTO, A.** *Adv. Drug Deliv. Rev.,* 1990, vol. 4, 171-207
- **MOHAMMAD KASAAI.** *Carbohydrate Polymers,* 2007, vol. 68, 477-488